(19) Europäisches Patentamt
European Patent Office
Office européen des brevets

(11) Veröffentlichungsnummer: **0 044 416**
**B1**

(12) # EUROPÄISCHE PATENTSCHRIFT

(45) Veröffentlichungstag der Patentschrift: **04.12.85**

(51) Int. Cl.⁴: **C 07 D 249/12**

(21) Anmeldenummer: **81104767.9**

(22) Anmeldetag: **22.06.81**

(54) **Triazolidin-3,5-dion-oxyalkylverbindungen und Verfahren zu ihrer Herstellung.**

(30) Priorität: **21.07.80 DE 3027596**

(43) Veröffentlichungstag der Anmeldung: **27.01.82 Patentblatt 82/4**

(45) Bekanntmachung des Hinweises auf die Patenterteilung: **04.12.85 Patentblatt 85/49**

(84) Benannte Vertragsstaaten: **DE FR GB IT**

(56) Entgegenhaltungen: **EP-A-0 016 956**

(73) Patentinhaber: **BAYER AG, Konzernverwaltung RP Patentabteilung, D-5090 Leverkusen 1 Bayerwerk (DE)**

(72) Erfinder: **Rottmaier, Ludwig, Bergstrasse 85, D-5068 Odenthal (DE)**
Erfinder: **Merten, Rudolf, Dr., Berta-von-Suttner-Strasse 55, D-5090 Leverkusen (DE)**

EP 0 044 416 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue (Alk)oxyalkyltriazolidin-3,5-dione und Verfahren zu ihrer Herstellung.

Es wurden (Alk)oxyalkyltriazolidin-3,5-dione der Formel

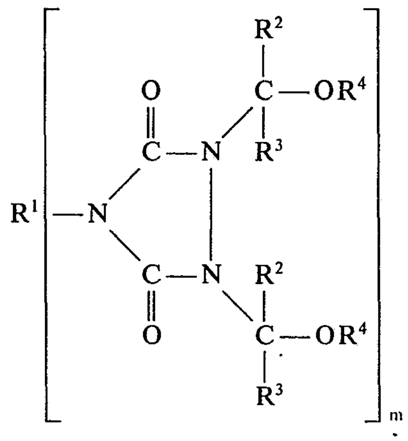

(I)

worin

m eine ganze Zahl von 1 bis 5 bedeutet,

$R^2$ und $R^3$ unabhängig voneinander Wasserstoff oder einen unsubstituierten aliphatischen Rest mit 1 bis 10 C-Atomen bedeuten,

$R^4$ für Wasserstoff, einen unsubstituierten aliphatischen Rest mit 1 bis 20 C-Atomen, mono- oder polycycloaliphatischen Rest mit 5 bis 10 C-Atomen oder araliphatischen Rest mit 7 bis 17 C-Atomen steht, und

$R^1$ für einen m-fach durch den Klammerausdruck besetzten, gegebenenfalls durch Halogen, Alkoxy oder Alkoxycarbonyl substituierten aliphatischen Rest mit insgesamt 1 bis 20 C-Atomen, mono- oder polycycloaliphatischen Rest mit insgesamt 5 bis 21 C-Atomen, araliphatischen Rest mit insgesamt 7 bis 17 C-Atomen, aromatischen Rest mit insgesamt 6 bis 20 C-Atomen, oder im Fall, wo m = 2 ist, weiterhin für eine gegebenenfalls in der genannten Weise substituierte Gruppe

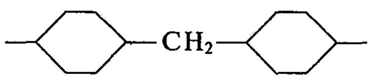 oder 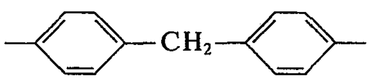

oder schließlich im Fall, wo m = 1 ist,
außerdem für den Rest

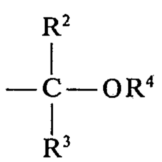

worin
$R^2$, $R^3$ und $R^4$ die obengenannte Bedeutung haben,
steht.

Als aliphatische Reste $R^1$ seien bevorzugt Reste mit 1 bis 12 C-Atomen, besonders bevorzugt Reste mit 1 bis 9 C-Atomen, genannt.

Als cycloaliphatische Reste $R^1$ seien bevorzugt Reste mit 6 bis 10 C-Atomen genannt.

Als araliphatische Reste $R^1$ seien bevorzugt Reste mit 7 bis 10 C-Atomen genannt.

Als aromatische Reste $R^1$ seien bevorzugt Reste mit 6 bis 14 C-Atomen, besonders bevorzugt Reste mit 6 bis 10 C-Atomen, genannt.

Als Substituenten von $R^1$ seien Halogen wie Fluor, Chlor, Brom, außerdem $C_1$—$C_{10}$-Alkoxy und -Alkoxycarbonyl genannt. Bevorzugt seien Chlor und $C_1$—$C_4$-Alkoxy genannt.

Für $R^2$ und $R^3$ seien, sofern sie aliphatische Reste sind, bevorzugt solche mit 1 bis 4 C-Atomen, wie Methyl, Ethyl, Propyl, Butyl, genannt. Besonders bevorzugt ist Wasserstoff.

Für $R^4$ seien, sofern er aliphatische Reste darstellt, bevorzugt solche mit 1 bis 12 C-Atomen, sofern er cycloaliphatische Reste darstellt, solche mit 5 bis 10 C-Atomen, sofern er araliphatische Reste darstellt, solche mit 7 bis 10 C-Atomen genannt. Besonders bevorzugt ist Wasserstoff, und sofern $R^4$ aliphatische Reste darstellt, solche mit 1 bis 4 C-Atomen, wie Methyl, Ethyl, Propyl, Butyl, und der Benzylrest.

m steht bevorzugt für die Zahl 1, 2 oder 3, besonders bevorzugt für die Zahl 1 und 2.

Unter den erfindungsgemäßen Verbindungen seien in einer ersten Variante bevorzugt Verbindungen der Formel I genannt, in denen

m die Zahl 1, 2 oder 3 bedeutet,
$R^1$ und $R^4$ die angegebene Bedeutung haben und
$R^2$ und $R^3$ unabhängig voneinander für Wasserstoff oder Methyl stehen.

Weiterhin seien in einer weiteren Variante bevorzugt Verbindungen der Formel I genannt, in denen

m und $R^1$ die angegebene Bedeutung haben,
$R^2$ und $R^3$ unabhängig voneinander für Wasserstoff oder Methyl stehen und
$R^4$ für Wasserstoff, einen $C_{1-12}$-Alkylrest, einen $C_{5-6}$-Cycloalkylrest oder einen araliphatischen Rest mit 7 bis 17 C-Atomen steht.

Besonders bevorzugt sind in der dritten Variante Verbindungen der Formel I genannt, in denen

m und $R^1$ die angegebene Bedeutung haben,
$R^2$ und $R^3$ Wasserstoff bedeuten und
$R^4$ für Wasserstoff, $C_{1-4}$-Alkyl, wie Methyl, Ethyl, Propyl, Butyl, Isobutyl, für Cyclohexyl oder Benzyl steht.

Weiterhin sind in einer vierten Variante besonders bevorzugt Verbindungen der Formel I genannt, in denen

m die Zahl 2 bedeutet,
$R^2$, $R^3$ und $R^4$ die für die dritte Variante angegebene Bedeutung haben und
$R^1$ für eine der Gruppen

$-(CH_2)_{2-6}-$ 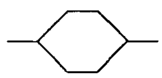 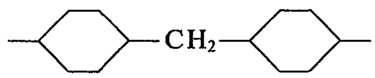

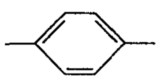 oder 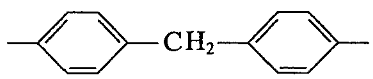

steht.

Außerdem bevorzugt sind noch in einer fünften Variante (Alk)Oxyalkyltriazolidin-3,5-dione der Formel

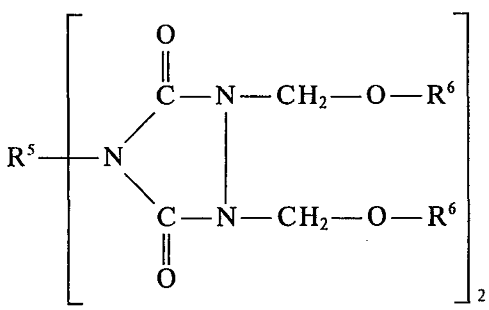 (Ia)

worin

$R^5$ für eine der Gruppen

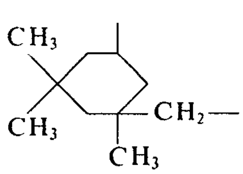 oder 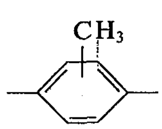 und

$R^6$ für Wasserstoff, $C_{1-4}$-Alkyl, Cyclohexyl oder Benzyl steht.

Es wurde weiterhin ein Verfahren zur Herstellung der (Alk)Oxyalkyl-triazolidin-3,5-dionen gefunden, das dadurch gekennzeichnet ist, daß man Triazolidin-3,5-dione der Formel

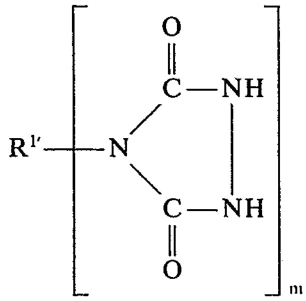

(II)

worin

m die angegebene Bedeutung hat und
$R^{1'}$ die für $R^1$ oder $R^6$ angegebenen Bedeutungen hat oder im Fall, wo m = 1 ist, auch für Wasserstoff stehen kann,

mit einem entsprechenden Keton oder Aldehyd der Formel

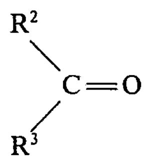

(III)

worin
$R^2$ und $R^3$ die angegebene Bedeutung haben,
gegebenenfalls in Gegenwart eines Katalysators umsetzt und gegebenenfalls das entstandene Hydroxyalkyltriazolidin-3,5-dion der Formel

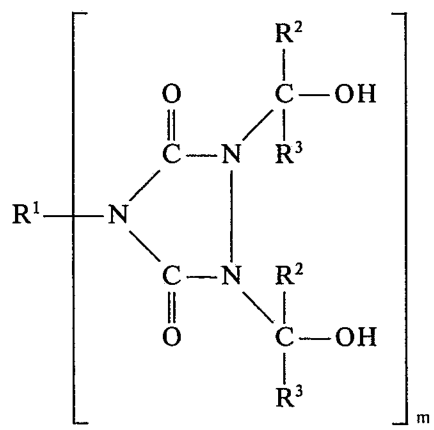

(IV)

worin
m, $R^1$, $R^2$ und $R^3$ die angegebene Bedeutung haben,
gegebenenfalls in Gegenwart eines Katalysators, mit einem Alkohol der Formel

$R^4OH$ (V)

worin
$R^4$ die angegebene Bedeutung hat,
umsetzt.

So können nach dem erfindungsgemäßen Verfahren Triazolidin-3,5-dione der Formel II mit Aldehyden oder Ketonen der Formel (III) zu Hydroxyalkyltriazolidin-3,5-dionen der Formel (IV) umgesetzt werden, die der Formel (I) mit der Bedeutung von $R^4 = H$ entspricht.

Diese können isoliert werden und in einer weiteren Reaktion mit den Alkoholen zu den Alkoxyalkyltriazolidin-3,5-dionen umgesetzt werden.

Die Hydroxyalkyltriazolidin-3,5-dione der Formel (IV) können aber auch ohne Isolierung in einem anschließenden Reaktionsschritt mit Alkoholen der Formel (V) zu den Alkoxyalkyltriazolidin-3,5-dionen

der Formel (I) umgesetzt werden.

Das erfindungsgemäße Verfahren kann aber auch so durchgeführt werden, daß Triazolidin-3,5-dione der Formel II mit Aldehyden oder Ketonen der Formel (III) und Alkoholen der Formel (V) gleichzeitig umgesetzt werden. In diesem Falle können Alkoxyalkyltriazolidin-3,5-dione der Formel (I) isoliert werden.

Bevorzugt wird das erfindungsgemäße Verfahren so durchgeführt, daß die Hydroxyalkyltriazolidon-3,5-dione der Formel (IV) hergestellt und gegebenenfalls mit Alkoholen der Formel (V) umgesetzt werden.

Zur Herstellung der neuen (Alk)Oxyalkyl-triazolidin-3,5-dione der Formel (I) kommen Triazolidin-3,5-dione der Formel (II) mit m = 1 in Betracht. Diese sind bekannt und können durch Reaktion von Hydrazincarbonsäureester mit Monoisocyanaten und nachfolgende Cyclisierung oder aus Hydrazin-N,N'-dicarbonsäurediamid durch Erhitzen auf 200°C erhalten werden [Liebigs Annalen der Chemie, Bd. 238, S. 41 (1894)].

Triazolidin-3,5-dione der Formel (II) die den Triazolidin-3,5-dion-Rest mehrfach enthalten (m größer als 1), können erhalten werden, indem man Amine der Formel

$$R^1(NH_2)_m \qquad (VI)$$

worin $R^1$ und m die angegebene Bedeutung haben,
mit Hydrazodicarbonamid oder mit 1,2,4-Triazolidin-3,5-dion bei 150 bis 280°C in Gegenwart oder Abwesenheit eines Lösungsmittels wie N-Methylpyrrolidon oder eines Lösungsmittelgemisches bei Drücken von 50 mbar bis 5 bar, gegebenenfalls in Gegenwart eines sauren oder basischen Katalysators wie eines Alkoholats oder eines tert. Amins, unter Abspaltung von Ammoniak, umsetzt.

Triazolidin-3,5-dione der Formel (II), die den Triazolidin-3,5-dion-Rest mehrfach enthalten (m größer als 1), können auch erhalten werden, wenn man Hydrazodicarbonamide der Formel

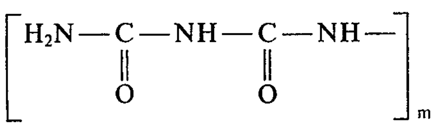

(VII)

worin $R^1$ und m die angegebene Bedeutung haben,
unter den obengenannten Bedingungen unter Ammoniakabspaltung cyclisiert.

N-monosubstituierte Hydrazodicarbonamide der Formel (VII) können analog bekannter Verfahren aus Semicarbazid und Isocyanaten der Formel (VIII)

$$R^1(NCO)_m \qquad (VIII)$$

worin $R^1$ und m die angegebene Bedeutung haben,
erhalten werden (Saunder, Frisch, »Polyurethanes« Part I, S. 205, Interscience Publishers 1962).

Als erfindungsgemäß einzusetzende Aldehyde oder Ketone der Formel (III) finden aliphatische Aldehyde oder Ketone Verwendung. Als Beispiele seien genannt: Formaldehyd, Acetaldehyd, Propionaldehyd, Butyraldehyd, 2-Ethylheptanal, Aceton, Butanon, Methyl-isopropylketon, Methyl-isobutylketon, Dipropylketon, 3-Methyl-5-heptanon und Dionylketon. Bevorzugt wird Formaldehyd, sowohl gasförmig oder in wäßriger Lösung, als auch in polymerer Form verwendet. Es können auch Mischungen der erfindungsgemäß einzusetzenden Oxo-Verbindungen eingesetzt werden.

Bei der Reaktion der Triazolidin-3,5-dione der Formel (II) mit Aldehyden oder Ketonen der Formel (III) können saure, basische oder neutrale Katalysatoren verwendet werden. Bevorzugt werden basische Katalysatoren eingesetzt, wie tertiäre Amine oder quaternäre Ammoniumsalze wie Triethylamin, Tri-n-butylamin, Triethanolamin, N,N'-Dimethylanilin, Urotropin, Tetramethylammoniumchlorid, Alkali- und Erdalkalihydroxide wie Natriumhydroxid, Bariumhydroxid, basisch reagierende Alkali- und Erdalkalisalze wie Natriumtetraborat, Lithiumcarbonat und Bariumcarbonat. Die Menge an Katalysator liegt zwischen 0,001 und 10%, bezogen auf die Reaktanten.

Prinzipiell kann die Umsetzung des Triazolidin-3,5-dions mit Aldehyden oder Ketonen auch ohne Zusatz von Katalysatoren durchgeführt werden, jedoch können dann höhere Temperaturen und/oder längere Reaktionszeiten erforderlich werden.

Die Reaktion der Triazolidin-3,5-dione der Formel (II) mit Aldehyden und Ketonen der Formel (III) kann mit stöchiometrischen Mengen an Aldehyden und Ketonen durchgeführt werden, d. h., 1 Mol Aldehyd oder Keton pro umsetzbare NH-Gruppe. Es kann auch ein Überschuß an Aldehyd oder Keton eingesetzt werden. Vorzugsweise wird mit stöchiometrischen Mengen gearbeitet.

Es kann jedoch auch weniger als 1 Mol Aldehyd oder Keton pro umsetzbare NH-Gruppe eingesetzt werden, so daß Produkte mit freien NH-Gruppen entstehen können. Diese freien NH-Gruppen können beim Einbau in Polymere nach bekannten Reaktionen weiterreagieren, so daß ganz spezielle modifizierte Kunststoffe erhalten werden können.

Bei der Reaktion der Triazolidin-3,5-dione der Formel (II) mit Aldehyden oder Ketonen der Formel (III)

können Lösungsmittel verwendet werden, es kann auch der Aldehyd oder das Keton (III) als Lösungsmittel dienen. Als Lösungsmittel dienen polare, gegebenenfalls mit Wasser mischbare organische Lösungsmittel. Bevorzugt seien genannt Wasser, Alkohole wie Methanol, Ethanol, n-Propanol, i-Propanol, n-Butanol, i-Butanole, Dimethylformamid, Dimethylacetamid, N-Methylpyrrolidon, Dimethylsulfon, Sulfolan.

Die Reaktion zwischen Triazolidin-3,5-dionen der Formel (II) und Aldehyden oder Ketonen der Formel (III) wird bei 10 bis 200°C, vorzugsweise bei 20 bis 150°C, gegebenenfalls unter erhöhtem Druck, durchgeführt.

Die Reaktionszeiten können sehr unterschiedlich sein. Sie können beispielsweise zwischen 30 Minuten und mehreren Tagen liegen. Durch entsprechende Wahl der Reaktionsbedingungen, z. B. Temperaturerhöhung und dadurch eventuell notwendige Druckerhöhung, oder aber nur durch Druckerhöhung können die Reaktionszeiten verkürzt werden.

Als erfindungsgemäß einzusetzende Alkohole der Formel (V) finden aliphatische, cycloaliphatische und araliphatische Alkohole Verwendung. Als Beispiele seien aufgeführt: Methanol, Ethanol, Iso- und n-Propanol, n-Butanol, Isobutanol, Amylalkohol, 1-Octanol, 1-Decanol, 1-Dodecanol, Stearylalkohol, Allylalkohol, Glykolmonomethylether, Cyclohexanol und Benzylalkohol. Besonders bevorzugt werden Methanol, Ethanol, Isopropanol, Butanol, Cyclohexanol und Benzylalkohol eingesetzt. Selbstverständlich können auch Mischungen der erfindungsgemäß einzusetzenden Alkohole verwendet werden.

Bei der Reaktion der Hydroxyalkyl-triazolidin-3,5-dione der Formel (IV) mit Alkoholen der Formel (V) können saure, basische oder neutrale Katalysatoren verwendet werden. Bevorzugt werden saure Katalysatoren eingesetzt. Besonders bevorzugt sind Mineralsäuren wie Salzsäure, Schwefelsäure, organische Säuren wie Oxalsäure, Essigsäure, Ameisensäure und Benzoesäure und Lewis-Säuren wie Bortrifluorid, Aluminiumfluorid und Zink(II)chlorid. Außerdem seien noch aufgeführt metallorganische Verbindungen wie Titan-tetrabutylat und Zinkoctoat.

Prinzipiell kann die Umsetzung der Hydroxyalkyl-triazolidin-3,5-dione mit den Alkoholen auch ohne Zusatz von Katalysatoren durchgeführt werden, jedoch können dann höhere Temperaturen und/oder längere Reaktionszeiten erforderlich werden.

Die Reaktion der Hydroxyalkyl-triazolidin-3,5-dione der Formel (IV) mit Alkoholen der Formel (V) kann mit stöchiometrischen Mengen Alkohol, d. h., 1 Mol Alkohol pro veretherbarer Hydroxylgruppe durchgeführt werden. Es kann auch ein Überschuß an Alkohol eingesetzt werden. Beim Arbeiten mit einem Überschuß wird dieser so gering wie möglich gehalten, insbesondere bei hochsiedenden Alkoholen.

Es kann auch weniger als 1 Mol Alkohol pro veretherbarer Hydroxyalkylgruppe eingesetzt werden, so daß Produkte mit freien Hydroxylgruppen entstehen. Solche hydroxygruppenhaltige (Alk)oxyalkyl-triazolidin-3,5-dione können auch entstehen, wenn die Reaktion zwischen Hydroxyalkyl-triazolidin-3,5-dionen und Alkoholen vorzeitig abgebrochen wird. Diese freien Hydroxylgruppen können beim Einbau in Polymere nach bekannten Reaktionen weiterreagieren, so daß spezielle, modifizierte Kunststoffe erhalten werden können.

Bei der Verwendung von mit Wasser nicht mischbaren Alkoholen kann der überschüssige Alkohol als Schleppmittel für das bei der Reaktion entstehende Wasser dienen. Selbstverständlich können jedoch auch andere mit Wasser nicht mischbare Lösungsmittel als Schleppmittel zur azeotropen Entfernung des bei der Reaktion entstehenden Wassers durch Azeotropdestillation dienen.

Insbesondere finden dafür aliphatische, araliphatische und aromatische Kohlenwasserstoffe, deren technische Gemische und deren Halogenierungsprodukte Verwendung. Als Beispiele seien genannt: Hexan, Octan, Cyclohexan, Toluol, Xylol, Solvesso 100, Methylenchlorid, Chloroform, Dichlorethan, Chlorbenzol.

Die Reaktion zwischen den Hydroxyalkyl-triazolidin-3,5-dionen und den Alkoholen wird im allgemeinen bei 30 bis 200°C, vorzugsweise bei 50 bis 150°C, gegebenenfalls unter erhöhtem Druck, durchgeführt.

Die Reaktionszeiten können sehr unterschiedlich sein. Sie können beispielsweise zwischen einer Stunde und mehreren Tagen liegen. Durch entsprechende Wahl der Reaktionsbedingungen, z. B. Temperaturerhöhung und dadurch eventuell notwendige Druckerhöhung, oder aber nur durch Druckerhöhung, können die Reaktionszeiten verkürzt werden.

Die Alkoxyalkyl-triazolidin-3,5-dione der Formel (I) sind wertvolle Ausgangsprodukte zur Herstellung von polymeren Verbindungen. Sie können in der Herstellung und Modifizierung von Phenolharzen, Harnstoff- und Melaminharzen eingesetzt werden.

Die nachfolgenden Beispiele sollen die Erfindung näher erläutern, wobei, wenn nicht anders angegeben, die Prozente Gewichtsprozente und Teile Gewichtsteile bedeuten.

## Beispiele

### Beispiel 1

Herstellung der Ausgangs-1,2,4-triazolidin-3,5-dione

a) Herstellung von 1,2,4-Triazolidin-3,5-dion

In einem 6-l-Dreihalskolben, der mit Rührer, Thermometer, Tropftrichter und Destillationsbrücke ausgestattet ist, werden 3 kg Sulfolan und 1,18 kg an der Luft getrocknetes Hydroazodicarbonamid innerhalb 2,5 Stunden auf 200°C aufgeheizt, wobei zu Beginn der Ammoniakentwicklung bei 150–160°C leichtes Vakuum angelegt wird. Dann wird die Temperatur innerhalb einer Stunde auf 210°C erhöht. Nach ca. 1,5 Stunden entsteht eine klare Lösung, die noch ca. 3,5 Stunden bei 210°C und 200 mbar bis zur Beendigung der Reaktion weitererhitzt wird. Das restliche Ammoniak wird nach Abkühlen auf 180°C bei 40 bis 80 mbar entfernt. Zu der abkühlenden Lösung werden bei Normaldruck 0,8 kg Toluol so zugetropft, daß praktisch kein Toluol abdestilliert. Das auskristallisierte, fast reine 1,2,4-Triazolidin-3,5-dion wird nach dem Abkühlen auf Raumtemperatur abgesaugt und mit Toluol gewaschen. Es werden 0,87 kg (= 86,2% d. Th.) getrocknetes 1,2,4-Triazolidin-3,5-dion mit einer Reinheit von 97,5%, bestimmt durch Titration mit n/10 Natronlauge gegen Phenolphthalein, erhalten.

b) Herstellung von 1,2-Ethandiyl-4,4'-bis-1,2,4-triazolidin-3,5-dion

600 g Hydrazodicarbonamid und 150 g Ethylendiamin werden in 500 ml N-Methylpyrrolidon 4 Stunden bei 175°C und 20 Stunden bei 200°C gerührt. Beim Abkühlen fällt ein Niederschlag aus, der abgesaugt und mit Ethanol gewaschen wird. Man erhält 462 g (80% der Theorie) 1,2-Ethandiyl-4,4'-bis-1,2,4-triazolidin-3,5-dion als farblose Kristalle vom Fp. >330°C.

c) Herstellung von 4,4'-Bis-(1,2,4-triazolidin-3,5-dion-4-yl)-dicyclohexylmethan

420 g 4,4'-Diaminodicyclohexylmethan und 472 g Hydrazodicarbonamid werden in 750 ml N-Methylpyrrolidon 4 Stunden bei 175°C, 8 Stunden bei 200°C und 4 Stunden bei 220°C gerührt. Das Reaktionsprodukt wird abgekühlt und anschließend in 3 l Wasser eingerührt. Dabei fällt ein Niederschlag aus, der abgesaugt und mit Wasser gewaschen wird. Man erhält 566 g (75% der Theorie) an 4,4'-Bis-(1,2,4-triazolidin-3,5-dion-4-yl)-dicyclohexylmethan als farblose Kristalle vom Fp. 305°C (Zersetzung).

d) Herstellung von 4-Phenyl-1,2,4-triazolidin-3,5-dion

Zu einer Lösung von 111,5 g Semicarbazidhydrochlorid in 700 g Wasser wird so lange in kleinen Portionen Soda zugesetzt, bis keine Gasentwicklung mehr zu beobachten ist.
Dann wird bei 40°C eine Lösung aus 119 g Phenylisocyanat in 100 g Aceton zugetropft. Zur Vervollständigung der Reaktion wird 2 Stunden bei 40°C nachgerührt und der gebildete Niederschlag durch Absaugen isoliert.
Der über Nacht an der Luft getrocknete Niederschlag wird in 300 g Sulfolan suspendiert und auf 205°C aufgeheizt, wobei ab 160°C durch Anlegen eines Wasserstrahlvakuums von 420 mbar das freiwerdende Ammoniak abgezogen wird. Nach einer Reaktionszeit von 5 Stunden wird bei einem Druck von 0,3 mbar das Lösungsmittel größtenteils entfernt und der verbliebende Rückstand aus n-Butanol umkristallisiert. Nach dem Absaugen und Trocknen werden 134 g 4-Phenyl-1,2,4-triazolidin-3,5-dion vom Fp. = 202–203°C (Lit. 203°C) erhalten.

### Beispiel 2

Zu 1220 g einer 37%igen wäßrigen Formaldehydlösung, der 5 g Natriumhydroxid zugesetzt wurden, werden unter Rühren 505 g Triazolidin-3,5-dion zugesetzt und auf 80°C erwärmt. Zur Vervollständigung der Reaktion wird noch eine Stunde bei 80°C gerührt. Durch Anlegen von Vakuum wird das vorhandene Wasser aus der klaren Lösung abdestilliert, wobei die Temperatur nicht über 50°C gesteigert wird. Es werden 945 g Tris-hydroxymethyltriazolidin-3,5-dion mit einem Wassergehalt von 0,5% (bestimmt durch Titration nach Fischer) und 45,71% abspaltbarem Formaldehyd (bestimmt nach saurer Destillation) erhalten. IR- und NMR-Spektrum sowie die Elementaranalyse bestätigen die angenommene Struktur.

berechnet für $C_5H_9N_3O_5$:
C = 31,4%, H = 4,71%, N = 22,0%, OH = 25,7%;

gefunden:
C = 31,2%, H = 4,9%, N = 22,1%, OH = 24,6%.

## Beispiel 3

50,5 g Triazolidin-3,5-dion werden in 122 g einer 37%igen wäßrigen Formaldehydlösung, der 0,5 g Borax zugesetzt waren, auf 80°C erwärmt und eine Stunde bei 80°C gerührt. Das Wasser wird im Vakuum bei 30 mbar und bei Temperaturen nicht über 50°C abdestilliert. Es werden 98,5 g Tris-hydroxymethyl-triazolidin-3,5-dion mit einem Wassergehalt von 4,8% und 42,83% abspaltbarem Formaldehyd erhalten.

## Beispiel 4

95,5 g des nach Beispiel 1 hergestellten Tris-hydroxymethyl-triazolidin-3,5-dions werden in 300 g n-Butanol gelöst und mit konzentrierter Schwefelsäure bis zu einem pH von 2 versetzt. Diese Lösung wird 2,5 Stunden auf Rückfluß erhitzt, wobei das frei werdende Wasser über einen Wasserabscheider entfernt wird. Nach dem Abkühlen wird etwas unlöslicher Rückstand durch Filtrieren entfernt und die erhaltene Lösung bei 60°C im Vakuum, zuletzt bei 0,3 mbar, von flüchtigen Bestandteilen befreit. Es werden 169 g rohes Tris-butoxymethyl-triazolidin-3,5-dion erhalten, welches zum Reinigen in Essigsäureethylester gelöst und diese Lösung mit verdünnter Natronlauge und dann mit Wasser ausgeschüttelt wird. Nach dem Abdestillieren des Lösungsmittels werden 151 g praktisch reines Tris-butoxymethyl-triazolidin-3,5-dion erhalten, dessen Struktur durch IR- und NMR-Spektrum sowie durch Elementaranalyse bewiesen wird.

berechnet für $C_{17}H_{33}N_3O_5$:
C = 57,7%, H = 9,18%, N = 11,2%;
gefunden:
C = 57,9%, H = 9,4%, N = 11,1%.

## Beispiel 5

95,5 g des nach Beispiel 1 hergestellten Tris-hydroxymethyl-triazolidin-3,5-dions, 162 g Benzylalkohol und 250 ml Toluol werden mit konzentrierter Schwefelsäure auf pH 2 eingestellt und 2,5 Stunden auf Rückfluß erhitzt, wobei das frei werdende Wasser über einen Wasserabscheider entfernt wird. Die abgekühlte Lösung wird durch Absaugen von etwas unlöslichen Rückstand befreit und im Vakuum eingeengt. Nach dem Trocknen bei einer Ölbadtemperatur von 130°C bei 0,3 mbar verbleiben 182 g einer öligen, hellgelben Flüssigkeit, welche zum Reinigen in Essigsäureethylester gelöst, mit 5%iger Natronlauge und dann mit Wasser ausgeschüttelt wird. Das Lösungsmittel wird am Rotationsverdampfer entfernt, wobei 176 g praktisch reines Tris-benzyloxymethyl-triazolidin-3,5-dion erhalten werden, dessen Struktur mittels IR- und NMR-Spektrum sowie der Elementaranalyse bewiesen wird.

berechnet für $C_{26}H_{27}N_3O_5$:
C = 67,7%, H = 5,85%, N = 9,12%;
gefunden:
C = 68,0%, H = 6,1%, N = 9,1%.

## Beispiel 6

Eine Lösung aus 191 g Tris-hydroxymethyl-triazolidin-3,5-dion aus Beispiel 1 in 500 ml Ethanol wird mit 4 g p-Toluolsulfonsäure versetzt und 12 Stunden bei Rückfluß gerührt. Die flüchtigen Bestandteile werden bei 60°C im Vakuum, zuletzt bei 0,3 mbar, entfernt. Es werden so 256 g eines rohen Tris-ethoxymethyl-triazolidin-3,5-dions erhalten, welches zum Reinigen in Essigsäureethylester gelöst, mit 5%iger Natronlauge, und dann mit Wasser ausgewaschen wird. Nach dem Entfernen des Lösungsmittels am Rotationsverdampfer verbleiben 173 g praktisch reines Tris-ethoxymethyl-triazolidin-3,5-dion, dessen Struktur durch IR- und NMR-Spektrum sowie durch Elementaranalyse bewiesen wird.

berechnet für $C_{11}H_{21}N_3O_5$:
C = 48,0%, H = 7,63%, N = 15,27%;
gefunden:
C = 47,8%, H = 7,5%, N = 15,4%.

## Beispiel 7

Zu 122 g einer 37%igen wäßrigen Formalinlösung, die 0,5 g Natriumtetraborat enthält, werden 50,5 g Triazolidin-3,5-dion zugesetzt und eine Stunde bei 80°C gerührt. Durch Anlegen von Vakuum bei Temperaturen bis 50°C wird der größte Teil des Wassers entfernt. Der viskose Rückstand wird in 300 ml Methanol gelöst und mit wfr. Natriumsulfat getrocknet. Die wasserfreie, methanolische Lösung wird mit 2 g p-Toluolsulfonsäure versetzt und unter Rückfluß gerührt. Nach 5stündigem Sieden wird die Reaktion abgebrochen und das Lösungsmittel im Vakuum, zuletzt bei 0,3 mbar, bei Temperaturen bis 45°C, entfernt. Es werden 103 g eines gelblichen, viskosen Rückstandes erhalten, der laut IR-Spektrum noch Hydroxylgruppen enthält. Die Analyse der Hydroxylgruppen erbrachte 7,9% OH-Gruppen, so daß ca. 2/3 der Hydroxymethyl- zu Methoxymethylgruppen reagiert haben. Auch das NMR-Spektrum bewies, daß ca. 2/3 Methoxymethyl- und ca. 1/3 Hydroxymethylgruppen entstanden waren.

## Beispiel 8

Zu 3900 g 37%iger wäßriger Formaldehydlösung, die 15 g NaOH enthält, werden bei Raumtemperatur 1515 g Triazolidin-3,5-dion unter Rühren portionsweise zugesetzt. Die entstandene Lösung wird eine Stunde bei 50°C gerührt, und durch Anlegen von Wasserstrahlvakuum wird die Hauptmenge des Wassers entfernt. Der entstehende hellgelbe viskose Rückstand wird durch Lösen in Aceton und nachfolgendes Abkühlen zum Kristallisieren gebracht. Es werden nach dem Absaugen und Trocknen des Niederschlags 2091 g (= 72,7% d. Th.) reines Tris-hydroxymethyl-triazolidin-3,5-dion (Fp = 109—110°C) erhalten, dessen Struktur mittels IR- und NMR-Spektrum sowie der Elementaranalyse bewiesen wird.

berechnet für $C_5H_9N_3O_5$:
C = 31,4%, H = 4,71%, N = 22,0%;
gefunden:
C = 31,5%, H = 4,7%, N = 21,9%.

## Beispiel 9

In einer Lösung von 0,7 g NaOH in 50 g Methanol werden bei Raumtemperatur 13,5 g Paraformaldehyd gelöst. Zu der erhaltenen klaren Lösung werden bei Raumtemperatur 22,8 g 1,2-Bis-(triazolidin-3,5-dion-4-yl)-ethan eingerührt und nach Beendigung der leicht exothermen Reaktion 1 h bei 30°C gerührt. Anderntags wird der voluminöse Niederschlag abgesaugt und getrocknet. Es werden 28 g praktisch reines 1,2-Bis-[1,2-bis-(hydroxymethyl)-triazolidin-3,5-dion-4-yl]-ethan (Fp = 165—167°C, gemessen auf einer Koflerbank) erhalten, dessen Struktur durch IR- und NMR-Spektrum sowie durch Elementaranalyse bewiesen wird.

berechnet für $C_{10}H_{16}N_6O_8$:
C = 34,5%, H = 4,6%, N = 24,1%;
gefunden:
C = 34,4%, H = 4,6%, N = 24,2%.

## Beispiel 10

Eine Mischung aus 660 g n-Butanol, 130 g Paraformaldehyd und 2,5 g Urotropin wird auf Rückfluß erhitzt bis eine klare Lösung entsteht. Nach dem Abkühlen auf Zimmertemperatur werden 378 g 4,4'-Bis-(triazolidin-3,5-dion-4-yl)-dicyclohexylmethan zugesetzt. Diese Suspension wird in 30 Minuten auf 100°C erhitzt, dann mit 1 g p-Toluolsulfonsäure versetzt und 6 Stunden unter azeotropen Rückfluß gehalten, wobei das entstehende Wasser über einen Wasserabscheider entfernt wird. Die abgekühlte Lösung wird mit Essigsäureethylester verdünnt, mit 5%iger Natronlauge und dann mit Wasser ausgewaschen, über Natriumsulfat getrocknet und im Vakuum, zuletzt bei 0,5 mbar und 50°C eingeengt. Es werden 718 g eines rohen 4,4'-Bis-[1,2-bis-(butoxymethyl)-triazolidin-3,5-dion-4-yl]-dicyclohexylmethans erhalten, dessen Struktur mittels IR- und NMR-Spektrum sowie der Elementaranalyse bewiesen wird.

berechnet für $C_{37}H_{66}N_6O_8$:
C = 61,5%, H = 9,1%, N = 11,6%;
gefunden:
C = 61,1%, H = 8,9%, N = 11,9%.

Beispiel 11

a) Zu 150 g 37%iger wäßriger Formaldehydlösung, welche 0,5 g 45%iger Natronlauge enthält, werden bei Raumtemperatur 93,2 g 1,2-Ethandiyl-4,4'-bis-1,2,4-triazolidin-3,5-dion unter Rühren zugesetzt. Die Temperatur steigt danach auf 45°C an. Die klare Lösung wird eine Stunde bei 50°C gerührt und durch Anlegen von Vakuum eingeengt. Der viskose Rückstand wird mit 250 g n-Butanol und 2,5 g konz. Salzsäure versetzt und langsam bis zum Rückfluß des Butanols erwärmt. Das noch vorhandene und das bei der Reaktion entstehende Wasser wird über einen Wasserabscheider entfernt. Nach Beendigung der Reaktion wird von etwas unlöslichem Rückstand filtriert, die Lösung mit 80 g Essigsäureethylester verdünnt und zweimal mit Wasser ausgewaschen. Die über Natriumsulfat getrocknete organische Phase wird im Vakuum zuletzt bei 0,4 mbar und 60°C bis zur Gewichtskonstanz eingeengt. Es wird ein viskoses, hellgelbes Öl erhalten, welches laut IR- und NMR-Spektren sowie der Elementaranalyse hauptsächlich aus 1,2-Ethandiyl-4,4'-bis-[bis-(n-butoxymethyl)-1,2,4-triazolidin-3,5-dion] besteht.

berechnet für $C_{26}H_{48}N_6O_8$:
C = 54,6%, H = 8,39%, N = 14,7%;
gefunden:
C = 54,4%, H = 8,8%, N = 15,1%.

b) 32,1 g eines Polyesters mit 5,3% Hydroxylgruppen, hergestellt durch Veresterung von Terephthalsäure, Ethylenglykol und Glycerin, werden in 32,1 g Ethylenglykolmonomethyletheracetat gelöst, mit 14,3 g des nach Beispiel 10a hergestellten Harzes und 0,46 g p-Toluolsulfonsäure versetzt und vermischt.

Ein mit dieser Lösung bestrichenes Eisenblech wird 20 Minuten bei 160°C erwärmt. Es resultiert ein fast farbloser Lackfilm mit glänzender Oberfläche und sehr guter Elastizität, der selbst beim Knicken des Eisenbleches nicht bricht.

Beispiel 12

Zu 32,5 g einer mit Natronlauge auf pH 9 eingestellten 37%igen Formaldehydlösung werden bei Raumtemperatur 35,4 g 4-Phenyl-triazolidin-3,5-dion eingerührt und eine Stunde bei 60°C gerührt. Nach dem Abdestillieren von Wasser im Vakuum werden 200 g n-Butanol und 0,5 g konz. Schwefelsäure zugesetzt und solange am Wasserabscheider unter azeotropem Rückfluß gerührt, bis kein Wasser mehr abdestilliert. Die erhaltene Lösung wird mit 50 g Essigsäureethylester versetzt, zweimal mit Wasser ausgeschüttelt und nach dem Trocknen über Natriumsulfat im Vakuum, zuletzt bei 0,4 mbar und 60°C, eingeengt. Es werden 67 g eines viskosen Öles erhalten, welches lt. IR- und NMR-Spektren sowie der Elementaranalyse hauptsächlich aus 1,2-Bis-(n-butoxymethyl)-4-phenyl-triazolidin-3,5-dion besteht.

berechnet für $C_{18}H_{27}N_3O_4$:
C = 61,8%, H = 7,74%, N = 12,03%;
gefunden:
C = 61,6%, H = 7,6%, N = 12,2%.

**Patentansprüche**

1. (Alk)Oxyalkyltriazolidin-3,5-dione der Formel

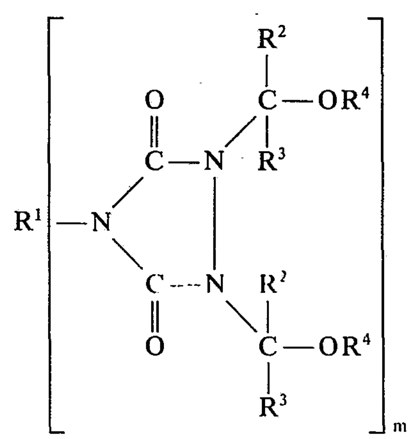

(I)

worin

m eine ganze Zahl von 1 bis 5 bedeutet,

$R^2$ und $R^3$ unabhängig voneinander Wasserstoff oder einen unsubstituierten aliphatischen Rest mit 1 bis 10 C-Atomen bedeuten,

$R^4$ für Wasserstoff, einen unsubstituierten aliphatischen Rest mit 1 bis 20 C-Atomen, mono- oder polycycloaliphatischen Rest mit 5 bis 10 C-Atomen oder araliphatischen Rest mit 7 bis 17 C-Atomen steht, und

$R^1$ für einen m-fach durch den Klammerausdruck besetzten, gegebenenfalls durch Halogen, Alkoxy oder Alkoxycarbonyl substituierten aliphatischen Rest mit insgesamt 1 bis 20 C-Atomen, mono- oder polycycloaliphatischen Rest mit insgesamt 5 bis 21 C-Atomen, araliphatischen Rest mit insgesamt 7 bis 17 C-Atomen, aromatischen Rest mit insgesamt 6 bis 20 C-Atomen, oder im Fall, wo m = 2 ist, weiterhin für eine gegebenenfalls in der genannten Weise substituierte Gruppe

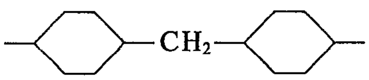 oder 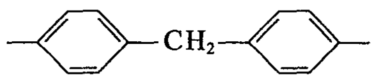

oder schließlich im Fall, wo m = 1 ist, außerdem für den Rest

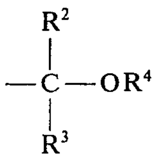

worin
$R^2$, $R^3$ und $R^4$ die obengenannte Bedeutung haben,
steht.

2. (Alk)Oxyalkyltriazolidin-3,5-dione nach Anspruch 1, dadurch gekennzeichnet, daß

m die Zahl 1, 2 oder 3 bedeutet,

$R^1$ und $R^4$ die angegebene Bedeutung haben und

$R^2$ und $R^3$ unabhängig voneinander für Wasserstoff oder Methyl stehen.

3. (Alk)Oxyalkyltriazolidin-3,5-dione nach Anspruch 2, dadurch gekennzeichnet, daß

m, $R^1$, $R^2$ und $R^3$ die angegebene Bedeutung haben und

$R^4$ für Wasserstoff, einen $C_{1-12}$-Alkylrest, einen $C_{5-6}$-Cycloalkylrest oder einen araliphatischen Rest mit 7 bis 17 C-Atomen steht.

4. (Alk)Oxyalkyltriazolidin-3,5-dione nach Anspruch 3, dadurch gekennzeichnet, daß

m und $R^1$ die angegebene Bedeutung haben,

$R^2$ und $R^3$ Wasserstoff bedeuten und

$R^4$ für Wasserstoff, $C_{1-4}$-Alkyl, Cyclohexyl oder Benzyl steht.

5. (Alk)Oxyalkyltriazolidin-3,5-dione nach Anspruch 4, dadurch gekennzeichnet, daß

m die Zahl 2 bedeutet,

$R^2$, $R^3$ und $R^4$ die angegebene Bedeutung haben und

$R^1$ für eine der Gruppen

$-(CH_2)_{2-6}-$ 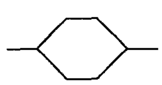 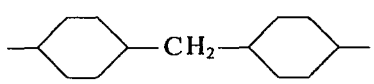

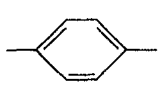 oder 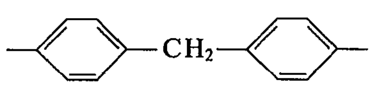

steht.

6. (Alk)Oxyalkyltriazolidin 3,5-dione der Formel

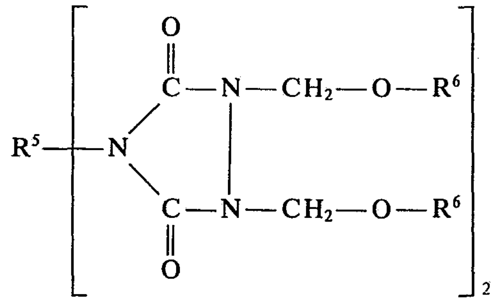

(Ia)

worin

$R^5$ für eine der Gruppen

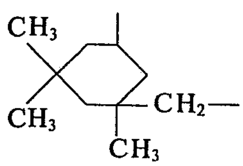 oder 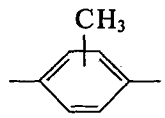 und

$R^6$ für Wasserstoff, $C_{1-4}$-Alkyl, Cyclohexyl oder Benzyl steht.

7. Verfahren zur Herstellung von (Alk)Oxyalkyltriazolidin-3,5-dionen nach einem der Ansprüche 1 oder 6, dadurch gekennzeichnet, daß man ein Triazolidin-3,5-dion der Formel

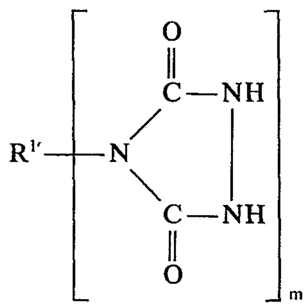

(II)

worin

m die angegebene Bedeutung hat und
$R^{1'}$ die für $R^1$ oder $R^6$ angegebenen Bedeutungen hat oder im Fall, wo $m = 1$ ist, auch für Wasserstoff stehen kann,

mit einem entsprechenden Keton oder Aldehyd der Formel

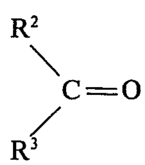

(III)

worin
$R^2$ und $R^3$ die angegebene Bedeutung haben,
gegebenenfalls in Gegenwart eines Katalysators umsetzt und gegebenenfalls das entstandene Hydroxyalkyltriazolidin-3,5-dion der Formel

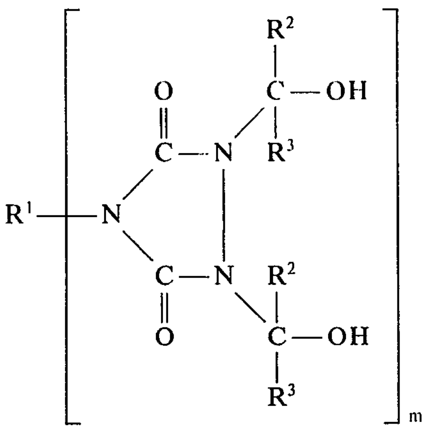

(IV)

worin
m, $R^1$, $R^2$ und $R^3$ die angegebene Bedeutung haben,
gegebenenfalls in Gegenwart eines Katalysators, mit einem Alkohol der Formel

$$R^4OH \quad (V)$$

worin
$R^4$ die angegebene Bedeutung hat,
umsetzt.

8. Verfahren nach Anspruch 7, dadurch gekennzeichnet, daß die bei der Reaktion der Triazolidin-3,5-dione (II) mit den Aldehyden oder Ketonen (III) entstandenen Hydroxyalkyltriazolidin-3,5-dione (IV) isoliert und gegebenenfalls in einer weiteren Reaktion mit den Alkoholen (V) umgesetzt werden.

9. Verfahren nach Anspruch 7, dadurch gekennzeichnet, daß die Triazolidin-3,5-dione (II) mit den Aldehyden oder Ketonen (III) und den Alkoholen (V) gleichzeitig umgesetzt werden.

10. Verfahren nach Anspruch 7, dadurch gekennzeichnet, daß die Umsetzung der Triazolidin-3,5-dione (II) bei einer Temperatur von 10 bis 200°C, gegebenenfalls in Gegenwart eines basischen Katalysators und gegebenenfalls in Gegenwart eines Lösungsmittels durchgeführt wird.

**Claims**

1. Alkoxyalkyl-/Hydroxyalkyl-triazolidine-3,5-diones of the formula

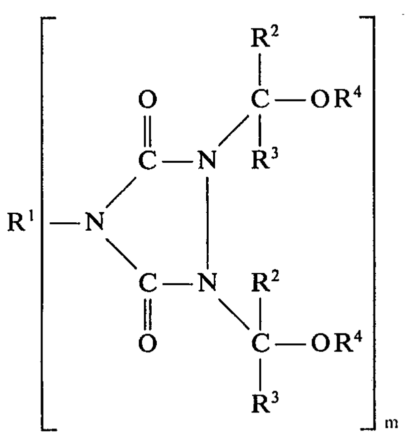

(I)

wherein

m denotes an integer from 1 to 5,
$R^2$ and $R^3$ independently of one another denote hydrogen or an unsubstituted aliphatic radical with 1 to 10 C atoms,
$R^4$ represents hydrogen, an unsubstituted aliphatic radical with 1 to 20 C atoms, mono- or polycycloaliphatic radical with 5 to 10 C atoms or araliphatic radical with 7 to 17 C atoms, and
$R^1$ represents an optionally halogen-, alkoxy- or alkoxycarbonyl-substituted aliphatic radical with a total of 1 to 20 C atoms, mono- or polycycloaliphatic radical with a total of 5 to

21 C atoms, araliphatic radical with a total of 7 to 17 C atoms, aromatic radical with a total of 6 to 20 C atoms, these radicals each carrying m of the structure in brackets, or if m = 2, also a group

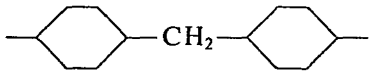 or 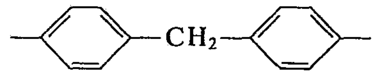

optionally substituted in the stated manner,
or finally, in the case where m = 1, also the radical

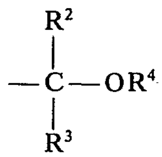

wherein
$R^2$, $R^3$ and $R^4$ have the abovementioned meaning.

2. Alkoxyalkyl-/Hydroxyalkyl-triazolidine-3,5-diones according to Claim 1, characterised in that

m denotes the number 1, 2 or 3,
$R^1$ and $R^4$ have the meaning given and
$R^2$ and $R^3$ independently of one another represent hydrogen or methyl.

3. Alkoxyalkyl-/Hydroxyalkyl-triazolidine-3,5-diones according to Claim 2, characterised in that

m, $R^1$, $R^2$ and $R^3$ have the meaning given and
$R^4$ represents hydrogen, a $C_{1-12}$-alkyl radical, a $C_{5-6}$-cycloalkyl radical or an araliphatic radical with 7 to 17 C atoms.

4. Alkoxyalkyl-/Hydroxyalkyl-triazolidine-3,5-diones according to Claim 3, characterised in that

m and $R^1$ have the meaning given,
$R^2$ and $R^3$ denote hydrogen and
$R^4$ represents hydrogen, $C_{1-4}$-alkyl, cyclohexyl or benzyl.

5. Alkoxyalkyl-/Hydroxyalkyl-triazolidine-3,5-diones according to Claim 4, characterised in that

m denotes the number 2,
$R^2$, $R^3$ and $R^4$ have the meaning given and
$R^1$ represents one of the groups

$-(CH_2)_{2-6}-$ 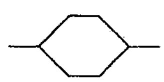 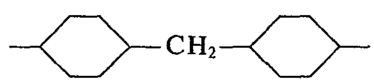

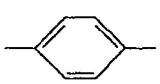 or 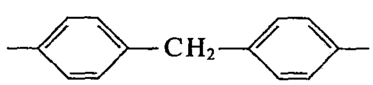

6. Alkoxyalkyl-/Hydroxyalkyl-triazolidine-3,5-diones of the formula

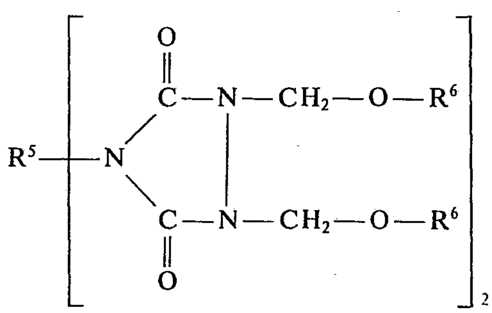

(Ia)

wherein

$R^5$ represents one of the groups

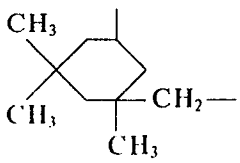 or 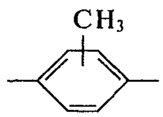 and

$R^6$ represents hydrogen, $C_{1-4}$-alkyl, cyclohexyl or benzyl.

7. Process for the preparation of alkoxyalkyl-/hydroxyalkyl-triazolidine-3,5-diones according to one of Claims 1 or 6, characterised in that a triazolidine-3,5-dione of the formula

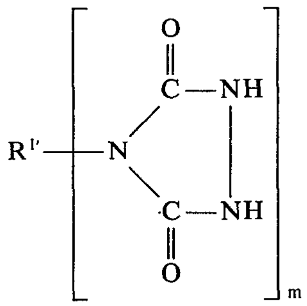

(II)

wherein

m has the meaning given and
$R^{1'}$ has the meanings given for $R^1$ or $R^6$ or in the case where m = 1, can also represent hydrogen,

is reacted with an appropriate ketone or aldehyde of the formula

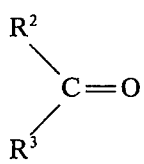

(III)

wherein
$R^2$ and $R^3$ have the meaning given,
if appropriate in the presence of a catalyst and, if desired, the hydroxyalkyltriazolidine-3,5-dione formed, of the formula

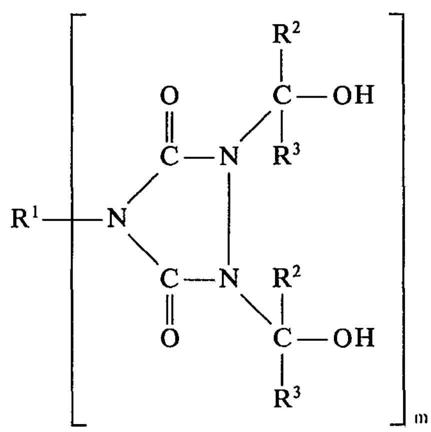

(IV)

wherein
m, $R^1$, $R^2$ and $R^3$ have the meaning given,
is reacted, if appropriate in the presence of a catalyst, with an alcohol of the formula

$R^4OH$ (V)

wherein

$R^4$ has the meaning given.

8. Process according to Claim 7, characterised in that the hydroxyalkyltriazolidine-3,5-diones (IV) formed in the reaction of the triazolindine-3,5-diones (II) with the aldehydes or ketones (III), are isolated and, if desired, reacted with the alcohols (V) in a further reaction.

9. Process according to Claim 7, characterised in that the triazolidine-3,5-diones (II) are reacted simultaneously with the aldehydes or ketones (III) and the alcohols (V).

10. Process according to Claim 7, characterised in that the reaction of the triazolidine-3,5-diones (II) is carried out at a temperature of 10 to 200°C, if appropriate in the presence of a basic catalyst and if appropriate in the presence of a solvent.

**Revendications**

1. Hydroxyalkyl- et alcoxyalkyl-triazolidine-3,5-diones de formule

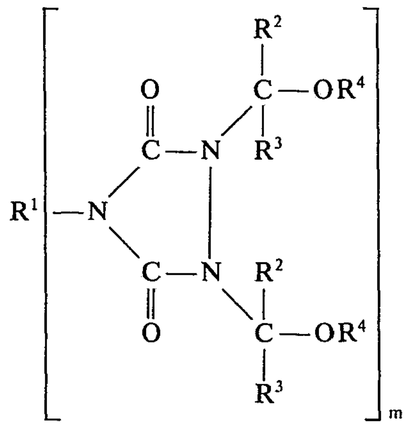

(I)

dans laquelle

m est un nombre entier de 1 à 5,
$R^2$ et $R^3$ représentent, indépendamment l'un de l'autre, l'hydrogène ou un reste aliphatique non substitué en $C_1$—$C_{10}$,
$R^4$ représente l'hydrogène, un reste aliphatique non substitué en $C_1$—$C_{20}$, un reste mono- ou poly-cycloaliphatique en $C_5$—$C_{10}$ ou un reste araliphatique en $C_7$—$C_{17}$, et
$R^1$ représente un reste, occupé m fois par l'expression entre parenthèses, aliphatique contenant au total 1 à 20 atomes de carbone, mono- ou poly-cycloaliphatique contenant au total 5 à 21 atomes de carbone, araliphatique contenant au total 7 à 17 atomes de carbone, aromatique contenant au total 6 à 20 atomes de carbone, éventuellement substitué par des halogènes, des groupes alcoxy ou alcoxycarbonyle, ou bien encore, lorsque m = 2, un groupe

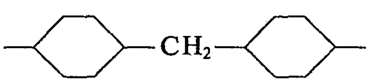 ou 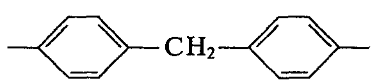

éventuellement substitué comme indiqué ci-dessus,
ou bien encore finalement, lorsque m = 1, le reste

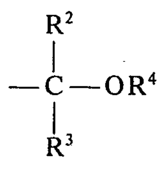

dans lequel $R^2$, $R^3$ et $R^4$ ont les significations indiquées ci-dessus.

2. Hydroxyalkyl- et alcoxyalkyl-triazolidine-3,5-diones selon la revendication 1, caractérisées en ce que:

m est égal à 1, 2 ou 3,
$R^1$ et $R^4$ ont les significations indiquées ci-dessus, et
$R^2$ et $R^3$ représentent, indépendamment l'un de l'autre, l'hydrogène ou un groupe méthyle.

3. Hydroxyalkyl- et alcoxyalkyl-triazolidine-3,5-diones selon la revendication 2, caractérisées en ce que:

m, $R^1$, $R^2$ et $R^3$ ont les significations indiquées ci-dessus, et
$R^4$ représente l'hydrogène, un groupe alkyle en $C_1$—$C_{12}$, cycloalkyle en $C_5$—$C_6$ ou un reste araliphatique en $C_7$—$C_{17}$.

4. Hydroxyalkyl- et alcoxyalkyl-triazolidine-3,5-diones selon la revendication 3, caractérisées en ce que:

m et $R^1$ ont les significations indiquées ci-dessus,
$R^2$ et $R^3$ représentent l'hydrogène, et
$R^4$ représente l'hydrogène, un groupe alkyle en $C_1$—$C_4$, cyclohexyle ou benzyle.

5. Hydroxyalkyl- et alcoxyalkyl-triazolidine-3,5-diones selon la revendication 4, caractérisées en ce que:

m est égal à 2,
$R^2$, $R^3$ et $R^4$ ont les significations indiquées ci-dessus, et
$R^1$ représente l'un des groupes

$-(CH_2)_{2-6}-$ 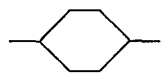 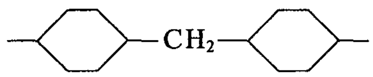

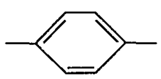 ou 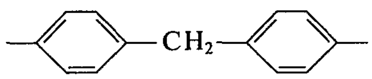

6. Hydroxyalkyl- et alcoxyalkyl-triazolidine-3,5-diones de formule

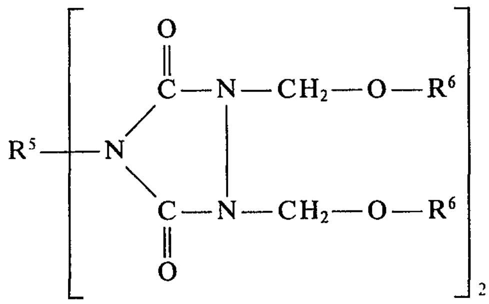

(Ia)

dans laquelle

$R^5$ représente l'un des groupes

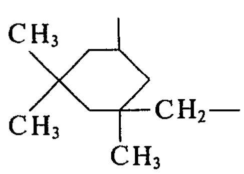 ou 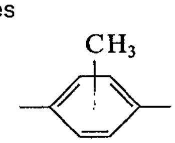 et

$R^6$ représente l'hydrogène, un groupe alkyle en $C_1$—$C_4$, cyclohexyle ou benzyle.

7. Procédé de préparation des hydroxyalkyl- et alcoxyalkyl-triazolidine-3,5-diones selon l'une des revendications 1 ou 6, caractérisé en ce que l'on fait réagir une triazolidine-3,5-dione de formule

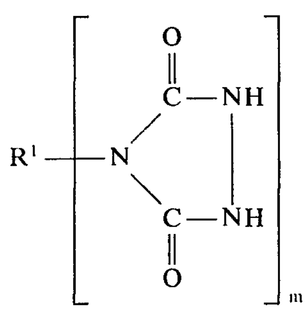

(II)

dans laquelle

m la signification indiquée ci-dessus et
$R^{1'}$ a les significations indiquées pour $R^1$ ou $R^6$, ou bien encore, dans le cas où m = 1, $R^{1'}$ peut représenter l'hydrogène,

avec une cétone ou un aldéhyde correspondant de formule

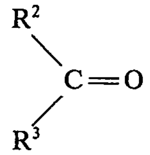

(III)

dans laquelle $R^2$ et $R^3$ ont les significations indiquées ci-dessus, éventuellement en présence d'un catalyseur, après quoi, le cas échéant, on fait réagir l'hydroxyalkyl-triazolidine-3,5-dione formée, de formule

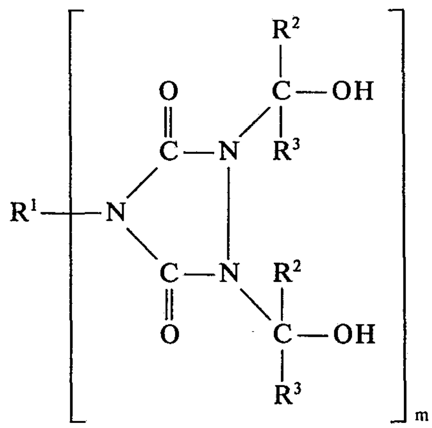

(IV)

dans laquelle m, $R^1$, $R^2$ et $R^3$ ont les significations indiquées ci-dessus, éventuellement en présence d'un catalyseur, avec un alcool de formule

$R^4OH$ (V)

dans laquelle $R^4$ a les significations indiquées ci-dessus.

8. Procédé selon la revendication 7, caractérisé en ce que l'on isole les hydroxyalkyl-triazolidine-3,5-diones IV formées dans la réaction des triazolidine-3,5-diones II avec les aldéhydes ou cétones III et on les fait réagir éventuellement dans une autre réaction avec des alcools V.

9. Procédé selon la revendication 7, caractérisé en ce que l'on fait réagir les triazolidine-3,5-diones II silmutanément avec les aldéhydes ou cétones III et les alcools V.

10. Procédé selon la revendication 7, caractérisé en ce que la réaction des triazolidine-3,5-diones II est effectuée à une température de 10 à 200°C, éventuellement en présence d'un catalyseur basique et éventuellement en présence d'un solvant.